(19) Europäisches Patentamt
European Patent Office
Office européen des brevets

(11) Veröffentlichungsnummer: **0 014 368**
**B1**

(12) # EUROPÄISCHE PATENTSCHRIFT

(45) Veröffentlichungstag der Patentschrift: **22.07.81**

(51) Int. Cl.³: **C 07 C 149/32**

(21) Anmeldenummer: **80100277.5**

(22) Anmeldetag: **21.01.80**

(54) **Verfahren zur Herstellung von 4-Nitrothioanisol.**

(30) Priorität: **30.01.79 DE 2903505**

(43) Veröffentlichungstag der Anmeldung: **20.08.80 Patentblatt 80/17**

(45) Bekanntmachung des Hinweises auf die Patenterteilung: **22.07.81 Patentblatt 81/29**

(84) Benannte Vertragsstaaten: **BE CH DE FR GB IT NL**

(73) Patentinhaber: **BAYER AG, Zentralbereich Patente, Marken und Lizenzen, D-5090 Leverkusen 1, Bayerwerk (DE)**

(72) Erfinder: **Hagemann, Hermann, Dr., Roggendorfstrasse 55, D-5000 Köln 80 (DE)**
Erfinder: **Klauke, Erich, Dr., Eichendorffweg 8, D-5068 Odenthal (DE)**
Erfinder: **Petruck, Gerd-Michael, Dr., Doerpfeldstrasse 49, D-4006 Erkrath 2 (DE)**

(56) Entgegenhaltungen:
**DE-A-275 037**
**BERICHTE DER DEUTSCHEN CHEMISCHEN GESELLSCHAFT, Band 42, 1909, Verlag Chemie GmbH, Weinheim, DE, BRAND: »Über o-Azothioanisol und o-Thiodianisidin«, Seiten 3463–3468**
**CHEMICAL ABSTRACTS, Band 47, Nr. 10, 25. Mai 1953, Zusammenfassung Spalte 4769d bis Spalte 4771f Columbus, Ohio, US, A. I. KIPRIANOV et al.: »Cyanine dyes which contain fluorine. III Cyanine dyes from derivates of 6-(trifluoromethylthio) benzothiazole«**
**CHEMICAL ABSTRACTS, Band 48, Nr. 9, 10. Mai 1954, Spalte 5005d, Columbus, Ohio, US, A. I. KIPRIANOV et al.: »Cyanine dyes which contain fluorine. II. Cyanine dyes from derivatives of 5-fluoro- and 5-trifluoromethylbenzothiazole«**
**JOURNAL OF THE AMERICAN CHEMICAL SOCIETY, Band 68, 16. März 1946, American Chemical Society, Gaston PA 18 042, US, PRICE et al.: »p-Nitrophenyl Disulfide, p-Nitrophenyl Sulfide and p-Nitrothiophenol«, Seiten 498–500**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



BUNDESDRUCKEREI BERLIN

Verfahren zur Herstellung von 4-Nitrothioanisol

Die Erfindung betrifft ein Verfahren zur Herstellung von 4-Nitrothioanisol durch Umsetzung von 4-Nitrochlorbenzol mit $Na_2S_2$, $Na_2S$ und einem Methylierungsmittel.

Es ist bekannt, 4-Nitrothiophenol herzustellen, indem man 4-Nitrochlorbenzol mit $Na_2S_2$ und NaOH nach folgender Reaktionsgleichung umsetzt.

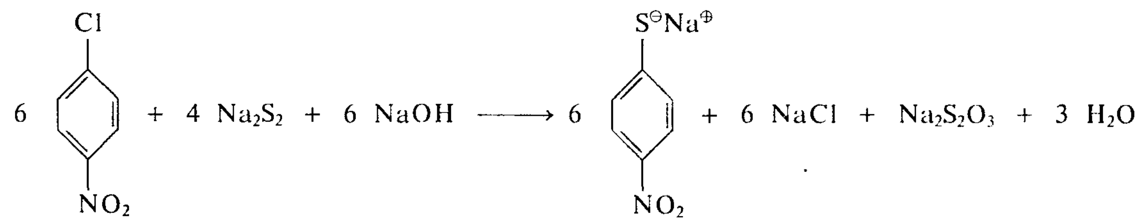


(C. C. Price und G. W. Stacy, Am. Soc. 68, 498 (1946))

Die Ausbeuten sind mit 60–65% angegeben. Durch Methylierung einer auf diesem Wege hergestellten Thiophenolatlösung geben Yagupolski und Kiprianov (J. Gen. Chem., USSR, 22, 2273–2277 [1952] ) für die Ausbeute an 4-Nitrothioanisol 75% bezogen auf 4-Nitrochlorbenzol an.

Weiterhin ist bekannt, daß sich bestimmte Nitrogruppen enthaltende organische Disulfide auch mit $Na_2S$ zu den Thiophenolaten reduzieren lassen, ohne daß die Nitrogruppe angegriffen wird (K. Brand, Chem. Ber., 42, 3463 [1909]).

Überraschenderweise wurde nun gefunden, daß man durch Kombination dieser an sich im Prinzip bekannten Einzelschritte in einer Eintopfreaktion ohne Isolierung von Zwischenstufen zu einer Ausbeute von 90% gelangt.

Gegenstand der Erfindung ist demnach ein Verfahren zur Herstellung von 4-Nitrothioanisol, das dadurch gekennzeichnet ist, daß man in einer Eintopfreaktion ohne Isolierung der Zwischenstufen 4-Nitrochlorbenzol nacheinander mit $Na_2S_2$, einer alkalischen $Na_2S$-Lösung und mit einem Methylierungsmittel umsetzt.

Man führt die Umsetzungen normalerweise in einem polaren organischen Lösungsmittel, wie z. B. einem Alkohol bzw. einer wäßrig-alkoholischen Mischung durch. Als Alkohol hat sich dabei besonders Methanol bewährt.

Die Temperatur der ersten beiden Reaktionsschritte sollte dabei zwischen 40 und 80°C, bevorzugt zwischen 50 und 70°C, liegen. Im allgemeinen wird eine Lösung von 4-Nitrochlorbenzol bei ca. 50°C vorgelegt und eine heiße methanolische Lösung von $Na_2S_2$ (aus $Na_2S \cdot 3\,H_2O$ und Schwefel hergestellt) zugegeben; es kann jedoch auch umgekehrt verfahren werden.

Der zweite Reaktionsschritt kann durch Zugabe einer Mischung aus Methanol, $Na_2S \cdot 3\,H_2O$ und methanolischer Natriummethylatlösung erfolgen. An Stelle der Natriummethylatlösung läßt sich auch eine konzentrierte wäßrige Alkalilauge, insbesondere NaOH einsetzen.

Außerdem hat es sich zur Erzielung einer guten Ausbeute als günstig erwiesen, im ersten Reaktionsschritt einen ca. 20%igen Überschuß an $Na_2S_2$ zu verwenden. Für den zweiten Reaktionsschritt werden dagegen nur etwa stöchiometrische Mengen nach folgender Reaktionsgleichung benötigt.

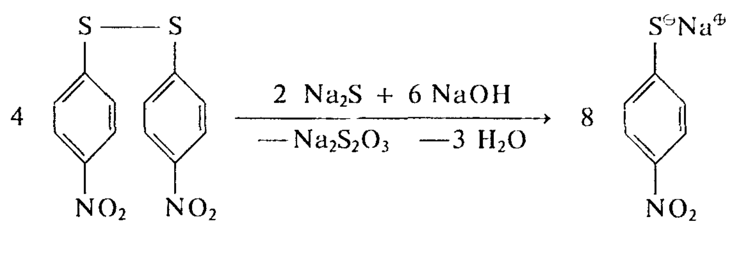


Die Methylierung läßt sich etwas über Raumtemperatur schon mit befriedigender Geschwindigkeit mit dem relativ schwachen Methylierungsmittel $CH_3Cl$ durchführen.

Methylchlorid bietet neben seiner technisch und wirtschaftlich guten Zugänglichkeit, z. B. gegenüber dem üblicherweise Verwendung findenden Dimethylsulfat den Vorteil einer geringeren Abwasserbelastung. Die Temperatur beträgt bei der Methylierung 27–35°C.

Es kann jedoch auch selbstverständlich zur Erhöhung der Reaktionsgeschwindigkeit und damit zur Verbesserung des Methylchloridumsatzes auch bei höherer Temperatur bzw. unter leichtem Überdruck gearbeitet werden.

4-Nitrothioanisol ist ein wichtiges Zwischenprodukt für die Herstellung eines hochwirksamen Insektizides folgender Struktur:

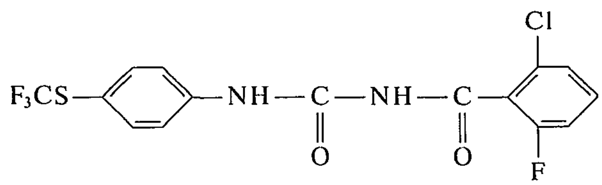

Eine über 90%ige Ausbeute ist vor allem deshalb außerordentlich vorteilhaft, weil bei einer Ausbeute um 75%, wie sie in der Literatur beschrieben ist, für die nächste Stufe, die Chlorierung, eine aufwendige Reinigungsoperation vorgenommen werden müßte. Das nach dem neuen Verfahren anfallende 4-Nitrothioanisol kann dagegen ohne eine weitere Reinigung chloriert werden.

Beispiel zur Herstellung des 4-Nitrothioanisols

395 g (2,5 Mol) 4-Nitrochlorbenzol und 700 ml Methanol werden vorgelegt und auf 50° C erwärmt. Dazu gibt man innerhalb von ca. 15 Minuten eine 60° heiße Lösung aus 198 g $Na_2S \cdot 3\,H_2O$ (1,5 Mol) und 48 g Schwefel (1,5 Mol) in 1900 ml Methanol und läßt ca. 2 Stunden bei Rückflußtemperatur nachreagieren.

Innerhalb von ca. 30 Minuten läßt man hierzu eine ebenfalls 60° C heiße Lösung aus 208 g 48%iger NaOH (2,5 Mol) und 82,5 g (0,625 Mol) $Na_2S \cdot 3\,H_2O$ in 300 ml Methanol tropfen und ca. 1 Stunde bei Rückflußtemperatur nachreagieren.

Es wird abgekühlt bei 27–35° C innerhalb von 2 Stunden 250 g (5 Mol) Methylchlorid eingeleitet, eine Stunde nachgerührt, 2000 ml Methanol im Wasserstrahlvakuum abdestilliert, 2000 ml Chlorbenzol zugegeben, 2000 ml Chlorbenzol/$H_2O$/$CH_2OH$ im Wasserstrahlvakuum abdestilliert, der Rückstand auf eine Nutsche gegeben und von den anorganischen Salzen abgetrennt. Der Filterrückstand wird mit 150 ml Chlorbenzol gewaschen, die gesammelten Filtrate eingeengt und der Rückstand destilliert. Man erhält 397 g (94% d. Th.) II, $Kp._{0,08}$: 105–107° C; Fp.: 68–70° C. Der nachdestillierbare Rückstand beträgt 9,5 g.

**Patentansprüche**

1. Verfahren zur Herstellung von 4-Nitrothioanisol, dadurch gekennzeichnet, daß man in einer Eintopfreaktion ohne Isolierung der Zwischenstufen 4-Nitrochlorbenzol nacheinander mit $Na_2S_2$, einer alkalischen $Na_2S$-Lösung und mit einem Methylierungsmittel umsetzt.
2. Verfahren nach Anspruch 1, dadurch gekennzeichnet, daß man in einem polaren Lösungsmittel oder Lösungsmittelgemisch arbeitet.
3. Verfahren nach Anspruch 2, dadurch gekennzeichnet, daß man als Lösungsmittel einen Alkohol, bevorzugt Methanol oder eine wäßrig-methanolische Mischung verwendet.
4. Verfahren nach Anspruch 1, dadurch gekennzeichnet, daß man in den ersten beiden Reaktionsschritten bei Temperaturen zwischen 40 und 80° C arbeitet.
5. Verfahren nach Anspruch 1, dadurch gekennzeichnet, daß man im ersten Reaktionsschritt mit ca. 20% Überschuß an $Na_2S_2$ arbeitet.
6. Verfahren nach Anspruch 1, dadurch gekennzeichnet, daß man als Methylierungsmittel Methylchlorid verwendet.

**Claims**

1. Process for the preparation of 4-nitrothioanisole, characterised in that 4-nitrochlorobenzene is successively reacted with $Na_2S_2$, an alkaline $Na_2S$ solution and a methylating agent, in a one-vessel reaction, without isolating the intermediate stages.
2. Process according to Claim 1, characterised in that it is carried out in a polar solvent or solvent mixture.
3. Process according to Claim 2, characterised in that the solvent used is an alcohol, preferably methanol, or an aqueous-methanolic mixture.
4. Process according to Claim 1, characterised in that the first two reaction steps are carried out at temperatures between 40 and 80° C.
5. Process according to Claim 1, characterised in that the first reaction step is carried out with about 20% excess of $Na_2S_2$.

6. Process according to Claim 1, characterised in that methyl chloride is used as the methylating agent.

**Revendications**

1. Procédé pour la préparation du 4-nitrothioanisole, caractérisé en ce que l'on fait réagir successivement le 4-nitrochlorobenzène avec $Na_2S_2$, une solution alcaline de $Na_2S$ et un agent de méthylation en une réaction en un seul récipient, sans isolement des intermédiaires.

2. Procédé selon la revendication 1, caractérisé en ce qu'on opère dans un solvant ou mélange solvant organique polaire.

3. Procédé selon la revendication 2, caractérisé en ce qu'on utilise comme solvant un alcool, de préférence le méthanol ou un mélange hydro-méthanolique.

4. Procédé selon la revendication 1, caractérisé en ce qu'on opère dans les deux premières étapes de réaction à des températures entre 40 et 80° C.

5. Procédé selon la revendication 1, caractérisé en ce qu'on opère dans la première étape de réaction avec un excès d'environ 20% de $Na_2S_2$.

6. Procédé selon la revendication 1, caractérisé en ce qu'on utilise comme agent de méthylation le chlorure de méthyle.